# EUROPEAN PATENT SPECIFICATION

(11) **EP 3 305 454 B1**
(45) Date of publication and mention of the grant of the patent: **05.11.2025**
(21) Application number: 17001567.1
(22) Date of filing: 21.09.2017
(51) Int. Cl.: B23K 9/32, A42B 3/28, A42B 3/04, A42B 3/22

(54) **WELDING HELMET CONFIGURATION PROVIDING REAL-TIME FUME EXPOSURE WARNING CAPABILITY**
SCHWEISSHELMKONFIGURATION MIT BEREITSTELLUNG VON ECHTZEITRAUCHEXPOSITIONSWARNUNGSFUNKTION
CONFIGURATION DE CASQUE DE SOUDAGE À CAPACITÉ D'AVERTISSEMENT D'EXPOSITION DES FUMÉES EN TEMPS RÉEL

(30) Priority: 30.09.2016 US 201615281784
(43) Date of publication of application: 11.04.2018
(73) Proprietor: Lincoln Global, Inc., Santa Fe Springs, CA 90670 (US)
(72) Inventor: Chantry, Bruce John, Solon, OH 44139 (US); Dunbar, Douglas N., Strongsville, OH 44136 (US)
(74) Representative: Grosse Schumacher Knauer von Hirschhausen

(56) References cited:
- WO-A1-2016/001651
- US-A1- 2012 286 958
- US-A1- 2017 013 904

## Description

### FIELD OF THE INVENTION

The invention is related to a welding system according to claim 1. Certain embodiments relate to the monitoring of fumes during a welding process. More particularly, certain embodiments relate to welding helmets, methods, and kits providing real-time fume exposure monitoring and warning capability.

### TECHNICAL BACKGROUND

During a welding process (e.g., an arc welding process), contaminants such as fumes can be generated which, if breathed in by a welder, can be harmful to the welder. In many welding situations (especially indoor situations), ventilation equipment is used to draw up and vent away the fumes, e.g. with a helmet described in US 2012/0286958 A1. However, sometimes the ventilation equipment may be inadequate for a particular welding process or scenario, or the ventilation equipment may not be properly set up or properly used by the welder. Because safety and health regulations tend to be performance based, compliance with industrial hygiene standards is dependent upon having actual workplace exposure determinations made by a qualified industrial hygienist. Current practice is to perform a full shift monitoring where a sampling device is placed on the worker to filter and collect a representative sample of the contaminants present in the worker's breathing zone. The goal is to obtain an eight hour time-weighted average concentration which may then be compared with the allowable levels in the regulations. However, many times, results cannot be obtained until several weeks after the sampling event as it is often necessary to send the samples to an accredited lab for analysis.

Further limitations and disadvantages of conventional, traditional, and proposed approaches will become apparent to one of skill in the art, through comparison of such approaches with embodiments of the present invention as set forth in the remainder of the present application with reference to the drawings.

### DESCRIPTION

In order to overcome the aforementioned problems and limitations, a welding system according to claim 1. Preferred embodiments are subject of the subclaims. Welding helmets, systems, and kits providing real-time fume exposure monitoring and warning capability during an arc welding process are disclosed herein. A welding helmet configured to protect the head of a user during a welding process is configured with an intelligent warning apparatus and an air-sampling pick-up and output port. The air-sampling pick-up and output port connects to a proximal end of an air sampling tube for sampling breathable air within the welding helmet, and a distal end of the air sampling tube connects to an air-sampling in-take port of an external aerosol monitoring device. The intelligent warning apparatus communicates with the aerosol monitoring device to receive air sample output data from the aerosol monitoring device, and to process the air sample output data to generate warning data and/or warning signals based on preset exposure level set points and/or exposure warning operating modes. As a result, a welder and/or, for example, a welder's supervisor can be readily informed of any unacceptable exposure to the welder during the welding process, before any significant harm can occur to the welder. Such helmets, systems, and kits may be used as a powerful day-to-day tool for both managing and more effectively understanding workplace exposures.

These and other features of the claimed invention, as well as details of illustrated embodiments thereof, will be more fully understood from the following description and drawings.

### BRIEF DESCRIPTION OF THE DRAWINGS

Fig. 1 is an illustration of a first example embodiment of a welding helmet for providing real-time fume exposure warning capability during a welding process;
Fig. 2 is an illustration of a second example embodiment of a welding helmet for providing real-time fume exposure warning capability during a welding process;
Fig. 3 is an illustration of a first example of a system for providing real-time fume exposure monitoring and warning capability using the welding helmet of Fig. 1 (or, alternatively, the welding helmet of Fig. 2) during a welding process;
Fig. 4 is a functional block diagram of the system of Fig. 3 showing functional elements of a first embodiment of the intelligent warning apparatus (IWA) of the welding helmet of Fig. 1 (or the welding helmet 200 of Fig. 2);
Fig. 5 is an illustration of a second example of a system for providing real-time fume exposure monitoring and warning capability using a slightly modified embodiment of a welding helmet during a welding process; and
Fig. 6 is a functional block diagram of the system of Fig. 5 showing the functional elements of the IWA of the welding helmet of Fig. 5.

### DETAILED DESCRIPTION

Embodiments of the present invention are capable of providing real-time fume exposure monitoring and warning capability during an arc welding process. In accordance with certain embodiments of the present invention, such capability is provided, at least in part, in a welding helmet worn by the user performing the welding process.

As used herein, the term "integrated" refers to being positioned on, being a physically integral part of, or being attached to (with or without the capability to be subsequently unattached). As used herein, the term "real-time" refers to the monitoring, communication, and processing of air sample output data during a welding process such that a welder and/or, for example, a welder's supervisor can be readily informed of any unacceptable exposure to the welder during the welding process, before any significant harm can occur to the welder. As used herein, the term "aerosol" means a system of particles dispersed in a gas (e.g., solid fume or smoke particles dispersed in air).

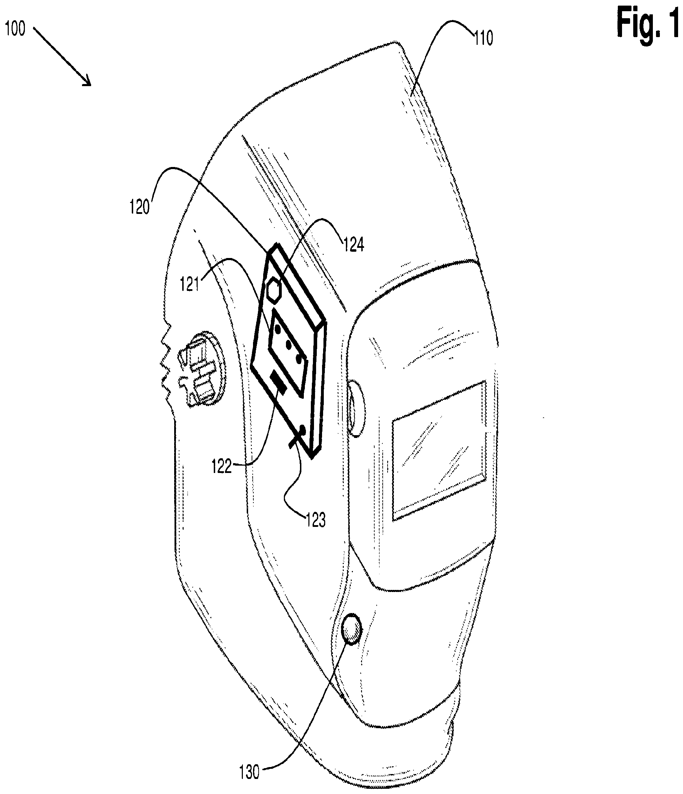

Details are described below herein with respect to Figs. 1-6. Fig. 1 is an illustration of a first example embodiment of a welding helmet 100 for providing real-time fume exposure warning capability during a welding process. The welding helmet 100 includes a welding headgear 110 configured to be worn on the head of a welder to protect the welder during a welding process. The welding helmet 100 also includes an intelligent warning apparatus (IWA) 120 integrated with the welding headgear 110. The IWA 120 is configured to communicatively interface with an external aerosol monitoring (EAM) device to receive air sample output data from the EAM device. The IWA 120 generates warning information and other environmental status information in response to receiving the air sample output data from the EAM device. Details of the interaction of the IWA 120 and the EAM device are described later herein.

In the embodiment of Fig. 1, the IWA 120 is positioned on the outside of the helmet 110. In accordance with certain embodiments of the present invention, the IWA 120 may be fixed on the welding headgear 110, or may be attachable to and detachable from the welding headgear 110. The IWA 120 includes a user interface 121, a communication input port 122 (e.g., a USB port), a radio frequency (RF) antenna 123, and an alarm or alert device 124. These elements of the IWA 120 are described in more detail later herein. In accordance with an alternative embodiment of the present invention, the alarm device 124 may be separate from the IWA 120 and integrated elsewhere on or within the helmet 100. In such an alternative embodiment, the IWA 120 would activate the alarm device 124 in a wired or wireless manner.

The welding helmet 100 further includes an air-sampling pick-up and output port (ASPOP) 130 integrated with the welding headgear 110. The ASPOP 130 is configured to sample or collect breathable air within the welding headgear 110 and to connect to a proximal end of an air sampling tube for funneling the sampled air away from the headgear 110. A distal end of the air sampling tube is attached to an EAM device as discussed later herein. As used herein, the term "breathable air" means a sample of air that is representative of the air being breathed by the welder while wearing the helmet 100. Even though the ASPOP 130 is shown near the front of the welding headgear 110 in Fig. 1, the ASPOP 130 may be located almost anywhere on the welding headgear 110 (e.g., toward the back of the welding headgear 110).

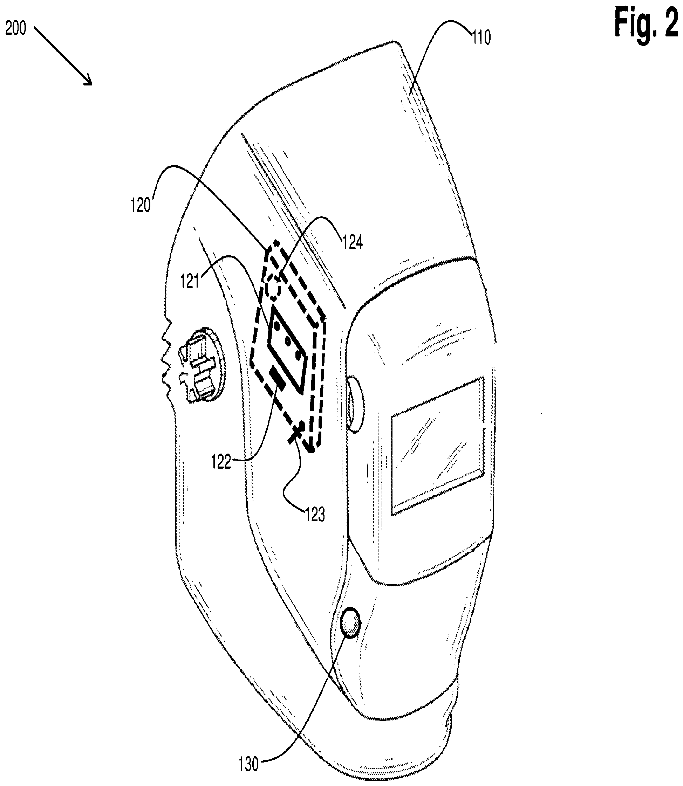

Fig. 2 is an illustration of a second example embodiment of a welding helmet 200 for providing real-time fume exposure warning capability during a welding process. The welding helmet 200 of Fig. 2 is similar to the welding helmet 100 of Fig. 1. However, the welding helmet 200 includes an IWA 120 that is largely integrated on the inside of the welding headgear 110, as indicated by the dashed lines of the IWA 120 in Fig. 2. However, the user interface 121, the communication input port 122, and the RF antenna 123 are still accessible from the outside of the welding headgear 110. The alarm or alert device 124 is on the inside of the welding headgear 110. In the embodiment of Fig. 2, much of the IWA 120 is protected by being integrated inside the welding headgear 110.

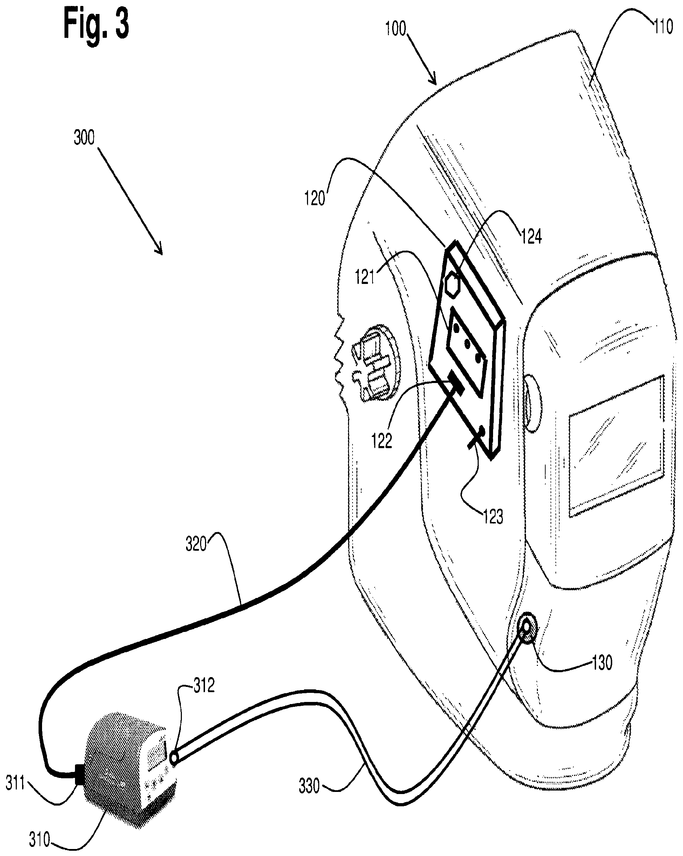

Fig. 3 is an illustration of a first example of a system 300 for providing real-time fume exposure monitoring and warning capability using the welding helmet 100 of Fig. 1 (or, alternatively, the welding helmet 200 of Fig. 2) during a welding process. In addition to the welding helmet 100, the system 300 includes an external aerosol monitoring (EAM) device 310 that is external to the helmet 100. The EAM device 310 is configured to determine the concentration of one or more contaminants in the sampled breathable air and generate associated air sample output data. For example, the EAM device 310 may be a laser photometer device. Examples of such laser photometer devices are the SIDEPAK^{™} devices as manufactured by TSI, Inc. The system 300 may further include a belt or harness (not shown) for holding the EAM device 310 while the belt or harness is worn by the user.

The system 300 also includes a communication cable 320 (e.g., a USB cable) connecting the communication input port 122 of the IWA 120 with a data output port 311 of the EAM device 310. During operation, the EAM device 310 sends air sample output data to the IWA 120 via the communication cable 320. Both the EAM device 310 and the IWA 120 may be powered by a battery or a re-chargeable power pack, for example. Alternatively, the EAM device 310 and/or the IWA 120 may be powered by, for example, an AC adapter that plugs into an electrical outlet, or by an auxiliary power source of a welding power source or wire feeder.

The system 300 further includes an air-sampling tube 330. The air-sampling tube 330 may be a durable, heat-resistant, flexible plastic tube, in accordance with an embodiment of the present invention. One end of the tube 330 connects to the ASPOP 130 on the helmet 100. The other end of the tube 330 connects to the air-sampling in-take port 312 on the EAM device 310. The EAM device 310 includes a pump (not shown) to create a vacuum which draws breathable air from within the helmet 100 through the ASPOP 130, through the tube 330, through the air-sampling in-take port 312, and into the EAM device 310 for analysis. In accordance with an embodiment of the present invention, the communication cable 320 and the air-sampling tube 330 are run together as one harness to provide a "clean" design implementation. In such an embodiment, the air-sampling in-take port 312 and the data output port 311 may be relatively close together on the EAM device 310. Similarly, the communication input port 122 and the ASPOP 130 may be relatively close together on the headgear 110.

During operation of the system 300, as the EAM device 310 draws in breathable air from the helmet 100, the EAM device 310 analyzes the sampled breathable air to determine particle mass concentration (e.g., via aerosol counting) in the sampled air. In accordance with an embodiment of the present invention, the EAM device 310 is a laser photometer device capable of determining real-time aerosol mass concentration and time-weighted average (TWA) aerosol mass concentration over longer periods of time, and is capable of discriminating between various ranges of particle size.

The mass concentrations determined by the EAM device 310 are sent, via the communication cable 320, to the IWA 120 as air sample output data for processing and analysis. Other forms or types of air sample output data may be generated by the EAM 310 and sent to the IWA 120 as well, in accordance with various embodiments of the present invention. For example, the EAM device 310 may also generate and log historical exposure data over time, or even over multiple welding process sessions.

The various elements of the system 300 of Fig. 3 (welding helmet, EAM device, IWA, air-sampling tube, communication cable, and harness) may be provided in the form of a kit which can be easily and readily assembled by a user for use, and subsequently disassembled after use.

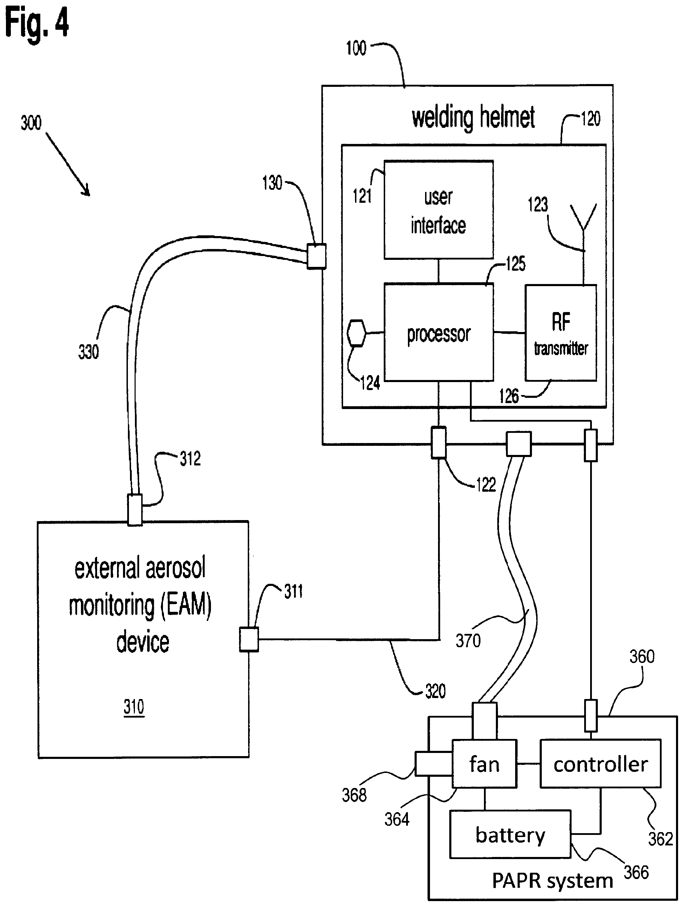

Fig. 4 is a functional block diagram of the system 300 of Fig. 3 showing functional elements of a first embodiment of the IWA 120 of the welding helmet 100 of Fig. I (or the welding helmet 200 of Fig. 2). As seen in Fig. 4, the IWA 120 includes a processor 125 and an RF transmitter 126 connected to the RF antenna 123. The processor 125 is operatively connected to the RF transmitter 126, the user interface 121, and the alarm or alert device 124. In accordance with various embodiments of the present invention, the processor 125 may be a software programmable microprocessor, a digital signal processor, a microcontroller, or any other processing device or chip capable of being configured or programmed to provide the processing functionality described herein for the processor 125. The processor 125 also operatively interfaces to the EAM device 310 via the communication cable 320 to receive air sample output data.

The processor 125 processes the air sample output data and generates exposure warning data and information. The exposure warning data and information may include alert data indicating when certain pre-defined exposure level limits are being exceeded, as well as other data and information including, for example, contaminant concentration level history and running time-averaged data.

The user interface 121 may be a touch-sensitive display or a configuration of switches and/or buttons, for example. Other types of user interfaces are possible as well. In one embodiment, the user interface is used by the welder to set one or more exposure level set points. An exposure level set point is a value that gets compared to the air sample output data (or a processed version thereof) by the processor 125. The air sample output data is representative of a concentration of one or more contaminants in the sampled breathable air. A user may rely on a standard maximum fume exposure guideline (MFEG) to determine an appropriate exposure level set point for a particular welding process, for example.

If the processor 125 determines that a concentration of a contaminant has been exceeded (i.e., determines that a warning condition has occurred), then the processor 124 may generate a warning signal to activate the alarm device 124 to alert the user of the situation. The alarm device 124 may be an audible alarm device, a visual alarm device, or a combination of the two, in accordance with various embodiments of the present invention. Other types of alarm devices are possible as well such as, for example, a vibrating alarm device. In accordance with an alternative embodiment of the present invention, the alarm device 124 may be separate from the helmet 100 and may be wirelessly activated by the IWA 120 of the helmet 100. Furthermore, if the welding helmet is of a type having an internal display that may be viewed by the welder, warning information from the IWA 120 may be displayed on the internal display.

In accordance with the invention, the user interface is used by the welder to select a pre-defined warning operating mode. A pre-defined warning operating mode is a mode of operation of the system 300 that is programmed or configured in the processor 125 which correlates to a particular welding process and the likely contaminants that may be produced as part of the that particular welding process. A particular pre-defined warning operating mode includes preset exposure level set points and possibly other parameters and/or operating algorithms corresponding to the particular welding process. A predefined warning operating mode is designed to keep the welder informed (e.g., via alerts) and safe with respect to contaminant exposure during that particular welding process.

In accordance with an embodiment of the present invention, the RF transmitter 126 receives warning data and information from the processor 125 and transmits the warning data and information to an external warning station (not shown). The external warning station may be, for example, a personal computer running an exposure software application in an office just outside a welding work environment in a factory. The external warning station may be manned by a welding supervisor to keep track of exposure levels of one or more welders currently working in the welding work environment.

In accordance with another embodiment of the present invention, the RF transmitter 126 receives warning data and information from the processor 125 and transmits associated command messages or signals to a welding power source (not shown) currently being operated by a user of the system 300 to turn off or shut down the welding power source, for example, when the processor determines that contaminant levels are unsafe or are approaching unsafe levels. Similarly, the RF transmitter 126 may transmit associated command messages or signals to a ventilation system (not shown) to change an operating state of the ventilation system (e.g., to increase a fan speed of the ventilation system) if the processor determines that contaminant levels are unsafe or are approaching unsafe levels.

The ventilation system is specific to an individual welder (e.g., a powered air purifying respirator (PAPR) 360 attached to the welding helmet 100). For purposes of illustration, a hard-wired PAPR system 360 is illustrated schematically in Fig. 4. As illustrated, the PAPR system 360 includes a controller 362, fan 364, and battery 366. That fan 364 draws air in through an intake 368 and expels the air through a hose 370 connected to the helmet 100. The controller 362 is operatively connected to the fan 364 to control operation (e.g., a speed) of the fan 364 based on a received input. In the embodiment of Fig. 4, the input is received from a hardwired connection from the processor 125 of the IWA 120. The battery 366 serves to power the fan 368 and controller 362.

The RF transmitter 126 may transmit command messages or signals to a controller 362 for the PAPR system 360 to automatically alter a fan speed or other property of the PAPR system 360. For PAPR systems 360 that are located on or near a user and/or connected to the welding helmet 100, the use of an RF transmitter 126 for communicating with the PAPR system 360 (RF receiver/transceiver of PAPR system 360 not illustrated) may be replaced by a hard-wired connection, as is illustrated in Fig. 4.

For PAPR systems 360, the fan 364 may operate at multiple speeds. For example, a high speed may be used when contaminate exposure is above a threshold and a low speed may be used when contaminate exposure is below the threshold. Therefore, based on the exposure information generated by the processor 125 of the IWA 120, the processor 125 may determine whether to send a signal to increase or decrease the fan speed to an appropriate level for the determined exposure. The threshold used for determining the fan speed may be different than that used for determining an alarm condition, and the processor may utilize respective different thresholds for different types of airborne contaminants. The thresholds may be set at the user interface 121 of the IWA 120. The user interface 121 of the IWA 120 may also serve as a user interface of the ventilation system, thereby allowing direct control of the fan or other properties of the system from the IWA 120.

For PAPR systems 360, the fan 364 is often powered by a battery 366 or other limited power source. Thus, controlling the fan speed to an appropriate level based on a determined exposure results in more efficient power consumption. For example, when exposure is low the fan does not need to operate at a high speed and, therefore, power consumption can be reduced by maintaining the fan at the low speed.

While the state of the ventilation or PAPR systems may be altered by changing the fan speed, any other properties of the systems may be changed in a similar manner so as to increase the system's efficiency.

In accordance with other embodiments of the present invention, the RF transmitter 126 may be replaced with some other type of wireless communication device such as, for example, an infrared transmitter or a sonic transmitter. Other types of wireless communication devices may be possible as well. Accordingly, the external warning station, the welding power source, and the ventilation system would be configured to communicate with such an alternative wireless communication device.

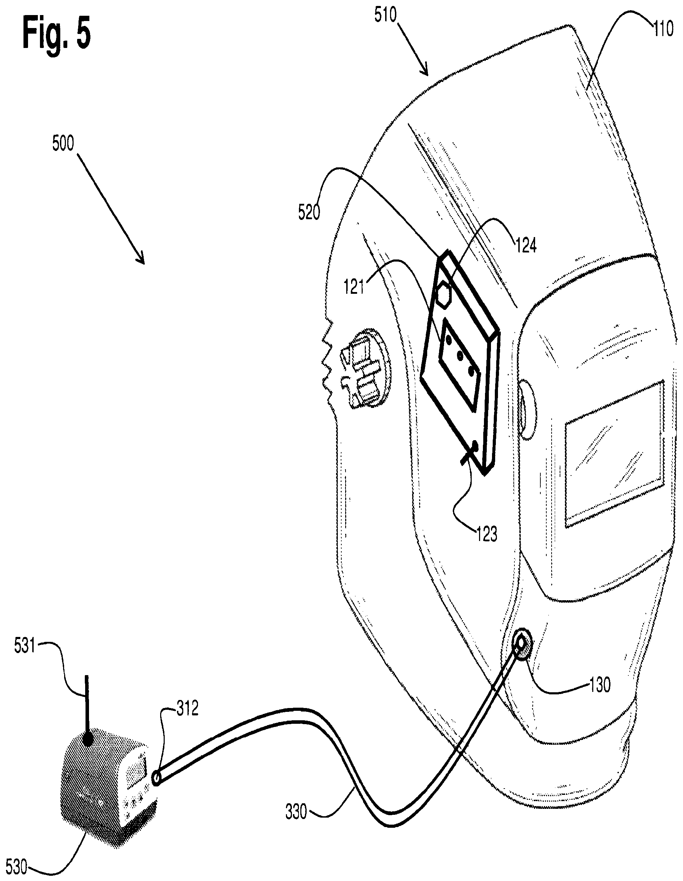

Fig. 5 is an illustration of a second example of a system 500 for providing real-time fume exposure monitoring and warning capability using a slightly modified embodiment of a welding helmet 510 during a welding process. In the system 500 of Fig. 5, communication between a second embodiment of an EAM device and a second embodiment of an IWA is performed via wireless means. The EAM device 530 of Fig. 5 does not have a data output port 311 but, instead, has an RF antenna 531 for transmitting air sample output data to the IWA 520 of Fig. 5. Similarly, the IWA 520 of Fig. 5 does not have a communication input port 122 but, instead, has the RF antenna 123 for receiving air sample output data from the EAM device 530. In accordance with alternative embodiments of the present invention, other wireless means of communicating air sample output data from the EAM device to the IWA may include infrared means, sonic means, or some other wireless means.

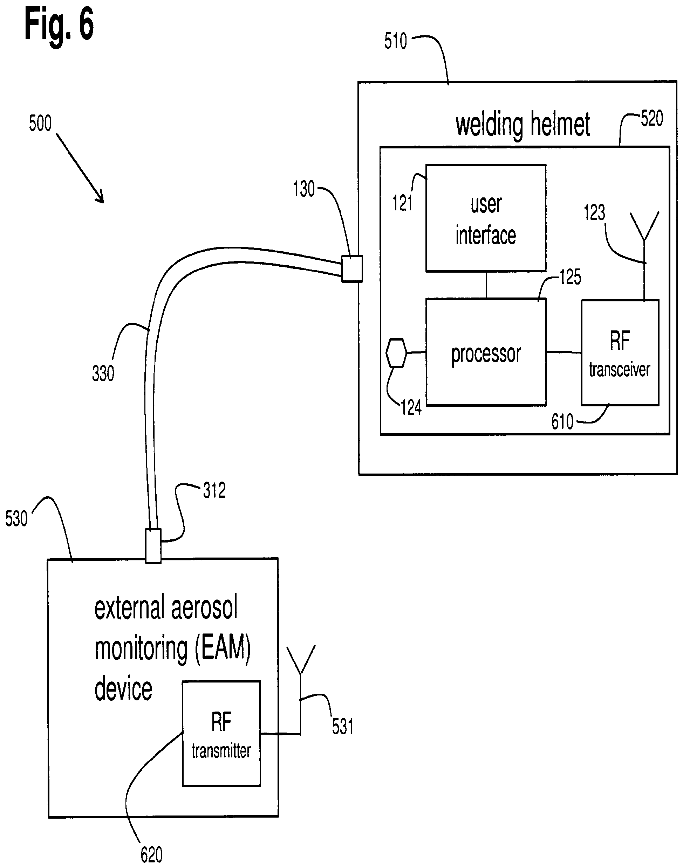

Fig. 6 is a functional block diagram of the system 500 of Fig. 5 showing the functional elements of the IWA 520 of the welding helmet 510 of Fig. 5. The EAM device 530 includes an RF transmitter 620 operatively connected to the RF antenna 531. The RF transmitter 620 functions to transmit air sample output data via the RF antenna 531 to the IWA 520. The IWA 520, instead of having an RF transmitter 126, has an RF transceiver 610 which functions to receive air sample output data via the RF antenna 123 and pass the air sample output data to the processor 125 for processing and analysis.

Also, the RF transceiver 610 functions to receive warning data and information from the processor 125 and transmit the warning data and information to an external warning station (not shown). The external warning station may be, for example, a mobile device running an exposure software application. The external warning station may be worn by an industrial hygienist inspecting a welding work environment in a factory to determine if exposure levels experienced by one or more welders currently working in the welding work environment meets current industrial standards.

Furthermore, the RF transceiver 610 may function to receive warning data and information from the processor 125 and transmit associated command messages or signals to a welding power source (not shown) currently being operated by a user of the system 500 to turn off or shut down the welding power source, for example, when the processor 125 determines that contaminant levels are unsafe or are approaching unsafe levels. Similarly, the RF transceiver 610 may transmit associated command messages or signals to a ventilation system (not shown) to change an operating state of the ventilation system, as described above, (e.g., to increase a fan speed of the ventilation system) if the processor 125 determines that contaminant levels are unsafe or are approaching unsafe levels. In accordance with other embodiments of the present invention, the RF transceiver 610 may be replaced with some other type of wireless communication device such as, for example, an infrared transceiver or a sonic transceiver. Other types of wireless communication devices may be possible as well. Accordingly, the external warning station, the welding power source, and the ventilation system would be configured to communicate with such an alternative wireless communication device.

In accordance with an embodiment of the present invention, the IWA is configured to generate and/or analyze historical exposure data. For example, the IWA may receive historical exposure data as a form of air sample output data from the EAM device and process and analyze the historical exposure data to generate statistical 531 to the IWA 520. The IWA 520, instead of having an RF transmitter 126, has an RF transceiver 610 which functions to receive air sample output data via the RF antenna 123 and pass the air sample output data to the processor 125 for processing and analysis.

Also, the RF transceiver 610 functions to receive warning data and information from the processor 125 and transmit the warning data and information to an external warning station (not shown). The external warning station may be, for example, a mobile device running an exposure software application. The external warning station may be worn by an industrial hygienist inspecting a welding work environment in a factory to determine if exposure levels experienced by one or more welders currently working in the welding work environment meets current industrial standards.

Furthermore, the RF transceiver 610 may function to receive warning data and information from the processor 125 and transmit associated command messages or signals to a welding power source (not shown) currently being operated by a user of the system 500 to turn off or shut down the welding power source, for example, when the processor 125 determines that contaminant levels are unsafe or are approaching unsafe levels. Similarly, the RF transceiver 610 may transmit associated command messages or signals to a ventilation system (not shown) to change an operating state of the ventilation system (e.g., to increase a fan speed of the ventilation system) if the processor 125 determines that contaminant levels are unsafe or are approaching unsafe levels.

In accordance with other embodiments of the present invention, the RF transceiver 610 may be replaced with some other type of wireless communication device such as, for example, an infrared transceiver or a sonic transceiver. Other types of wireless communication devices may be possible as well. Accordingly, the external warning station, the welding power source, and the ventilation system would be configured to communicate with such an alternative wireless communication device.

In accordance with an embodiment of the present invention, the IWA is configured to generate and/or analyze historical exposure data. For example, the IWA may receive historical exposure data as a form of air sample output data from the EAM device and process and analyze the historical exposure data to generate statistical exposure parameters and trend data as a form of exposure warning data and information. Such statistical exposure parameters and trend data may provide great insight to an industrial hygienist with respect to the environmental operation of a plant or factory (e.g., over a full eight-hour shift, or over an entire week).

In summary, an embodiment of the present invention comprises a welding helmet providing real-time fume exposure warning capability. The welding helmet includes a welding headgear configured to be worn on a head of a user to protect the user during a welding process. The welding helmet further includes an intelligent warning apparatus integrated with the welding headgear and configured to communicatively interface with an external aerosol monitoring device to receive air sample output data from the external aerosol monitoring device. The welding helmet also includes an air-sampling pick-up and output port integrated with the welding headgear and configured to connect to a proximal end of an air sampling tube for sampling breathable air within the welding headgear. A distal end of the air sampling tube is configured to connect to an air-sampling intake port of the external aerosol monitoring device. The intelligent warning apparatus includes a processor configured to process the air sample output data received from the external aerosol monitoring device to generate at least warning data. The intelligent warning apparatus also includes a user interface operatively connected to the processor and configured to allow a user to set at least one exposure level set point and/or to select a warning operating mode from a plurality of pre-defined warning operating modes. The intelligent warning apparatus may further include a wireless communication device operatively connected to the processor and configured to receive at least the warning data from the processor and to wirelessly transmit at least the warning data to an external warning station. The intelligent warning apparatus may also include a wireless communication device operatively connected to the processor and configured to wirelessly receive the air sample output data from the external aerosol monitoring device ay be configured to communicatively interface with a welding power source to operatively shut down the welding power source in response to the warning data. The intelligent warning apparatus may further include an alarm device or an alert device operatively connected to the processor and configured to alarm upon reception of a warning signal from the processor. In accordance with an alternative embodiment of the present invention, the alarm or alert device is not part of the intelligent warning apparatus but, instead, operatively interfaces to the intelligent warning apparatus to receive an activating warning signal from the intelligent warning apparatus.

A kit not in accordance with the invention providing real-time welding fume exposure monitoring and warning capability may include a welding helmet having an air-sampling pick-up and output port integrated therewith. The helmet is configured to be worn on a head of a user of the kit to protect the user during a welding process. The kit further includes an aerosol monitoring device having an air-sampling intake port and configured to generate air sample output data. The kit also includes an intelligent warning apparatus configured to communicatively interface with the aerosol monitoring device and to generate at least warning data in response to the air sample output data. The intelligent warning apparatus may be physically integrated with the welding helmet, or may be capable of being attached and detached from the welding helmet. The kit further includes an air-sampling tube having a proximal end and a distal end. The air-sampling tube is configured to operatively mate with the air-sampling pickup and output port of the welding helmet at the proximal end of the air-sampling tube, and further configured to operatively mate with the air-sampling intake port of the aerosol monitoring device at the distal end of the air-sampling tube. The kit may further include a communication cable having a proximal end and a distal end. The communication cable is configured to operatively mate with a communication input port of the intelligent warning apparatus at the proximal end of the communication cable. The communication cable is also configured to operatively mate with a data output port of the aerosol monitoring device at the distal end of the communication cable. Alternatively, the communication cable facilitates wired communication from the aerosol monitoring device to the intelligent warning apparatus. The intelligent warning apparatus and the aerosol monitoring device may be configured to wirelessly communicate with each other. The intelligent warning apparatus may include a wireless communication capability configured to transmit warning information generated by the intelligent warning apparatus to an external warning station. The intelligent warning apparatus may be configured to communicatively interface with an external ventilation system to effect a change in an operational state of the external ventilation system in response to the warning data. The intelligent warning apparatus may be configured to communicatively interface with a welding power source to operatively shut down the welding power source in response to the warning data. The intelligent warning apparatus may include a user interface configured to allow a user to set at least one exposure level set point and/or to select a warning operating mode from a plurality of pre-defined warning operating modes. The intelligent warning apparatus may include and alarm device or an alert device configured to be activated when a warning condition is determined by the intelligent warning apparatus. In accordance with an alternative embodiment of the present invention, the alarm or alert device is not part of the intelligent warning apparatus but, instead, operatively interfaces to the intelligent warning apparatus to receive an activating warning condition signal from the intelligent warning apparatus. The kit may further include a harness or belt for holding the aerosol monitoring device while the harness or belt is worn by the user.

While the claimed subject matter of the present application has been described with reference to certain embodiments, it will be understood by those skilled in the art that various changes may be made and equivalents may be substituted without departing from the scope of the claimed subject matter. In addition, many modifications may be made to adapt a particular situation or material to the teachings of the claimed subject matter without departing from its scope. Therefore, it is intended that the claimed subject matter not be limited to the particular embodiments disclosed, but that the claimed subject matter will include all embodiments falling within the scope of the appended claims.

**REFERENCE NUMBERS**

| | | | |
|---|---|---|---|
| 100 | welding helmet | 320 | cable |
| 110 | welding headgear | 330 | air-sampling tube |
| 120 | intelligent warning apparatus | 360 | powered are purifying respire tor / PAPR |
| 121 | user interface IWA | | |
| 122 | input port | 362 | controller |
| 123 | antenna | 364 | fan |
| 124 | alarm device / alert device | 366 | battery |
| 125 | processor | 368 | fan |
| 126 | transmitter | 370 | hose |
| 130 | air-sampling pich-up and output port / ASPOP | 500 | system |
| | | 510 | welding helmet |
| 200 | welding helmet | 520 | user interface IWA |
| 300 | system | 530 | device |
| 310 | device / external aerosol monitoring EAM | 531 | antenna |
| | | 610 | transceiver |
| 311 | data output port | 620 | transmitter |
| 312 | air-sampling in-take port | | |

## Claims

1. A system (300, 500) comprising:
a helmet (100, 200, 510) configured to be worn on a head of a user to protect the user;
the helmet comprising:
a welding headgear (110) configured to be worn on a head of a user to protect the user during a welding process;
an intelligent warning apparatus (120, 520) integrated with the welding headgear (110);
a ventilation system;
an external aerosol monitoring device (310) operatively coupled to the helmet (100, 200, 510); and
said intelligent warning apparatus configured to:
communicatively interface with the external aerosol monitoring device (310) to receive air sample output data from said external aerosol monitoring device;
the intelligent warning apparatus (120, 520) being configured to communicate with the ventilation system, thereby controlling at least one property of the ventilation system,
wherein said intelligent warning apparatus (120, 520) includes a processor (125) configured to process said air sample output data received from said external aerosol monitoring device to generate a signal used for controlling the at least one
property of the ventilation system,
wherein said intelligent warning apparatus (120, 520) further includes a user interface (121) operatively connected to said processor (125) and configured to allow a user to select a predefined warning operation mode including present exposure level set points and correlating to a particular welding process by including parameters corresponding to said particular welding process,
wherein said user interface (121) is configured to allow a user to a set an exposure threshold, wherein a state of the at least property is controlled based on the exposure threshold, and wherein the threshold is dependent on a type of airborne contaminate,
**characterized in that**
the ventilation system is a powered air purifying respirator (360) for the welding helmet (100, 200, 510).

2. The system (300, 500) of claim 1, wherein the at least one property of the ventilation system is a fan speed.

## Patentansprüche

1. System (300, 500), umfassend:
einen Helm (100, 200, 510), der dazu eingerichtet ist, auf dem Kopf eines Benutzers getragen zu werden, um den Benutzer zu schützen;
wobei der Helm umfasst:
eine Schweiß-Kopfhaube (110), die dazu eingerichtet ist, auf dem Kopf eines Benutzers getragen zu werden, um den Benutzer während eines Schweißprozesses zu schützen;
eine intelligente Warnvorrichtung (120, 520), die in die Schweiß-Kopfhaube (110) integriert ist;
ein Belüftungssystem;
eine externe Aerosol-Überwachungsvorrichtung (310), die mit dem Helm (100, 200, 510) wirkgekoppelt ist; und
wobei die intelligente Warnvorrichtung dazu eingerichtet ist:
mit der externen Aerosol-Überwachungsvorrichtung (310) kommunikativ zusammenzuwirken, um Luftprobenausgabedaten von der externen Aerosol-Überwachungsvorrichtung zu empfangen;
wobei die intelligente Warnvorrichtung (120, 520) dazu eingerichtet ist, mit dem Belüftungssystem zu kommunizieren, wodurch mindestens eine Eigenschaft des Belüftungssystems gesteuert wird,
wobei die intelligente Warnvorrichtung (120, 520) einen Prozessor (125) aufweist, der dazu eingerichtet ist, die von der externen Aerosol-Überwachungsvorrichtung empfangenen Luftprobenausgabedaten zu verarbeiten, um ein Signal zu generieren, das zum Steuern der mindestens einen Eigenschaft des Belüftungssystems verwendet wird,
wobei die intelligente Warnvorrichtung (120, 520) des Weiteren eine Benutzerschnittstelle (121) aufweist, die mit dem Prozessor (125) wirkgekoppelt ist und dazu eingerichtet ist, es einem Benutzer zu erlauben, einen vordefinierten Warnbetriebsmodus auszuwählen, der momentane Expositionsniveau-Sollwerte enthält und mit einem speziellen Schweißprozess korreliert, indem er Parameter enthält, die dem speziellen Schweißprozess entsprechen,
wobei die Benutzerschnittstelle (121) dazu eingerichtet ist, es einem Benutzer zu erlauben, eine Expositionsschwelle einzustellen, wobei ein Zustand der mindestens einen Eigenschaft auf der Grundlage der Expositionsschwelle gesteuert wird und wobei die Schwelle von einer Art von luftverunreinigenden Schwebeteilchen abhängt,
**dadurch gekennzeichnet, dass**
das Belüftungssystem ein elektrisch betriebenes Luftreinigungs-Atemschutzgerät (360) für den Schweißhelm (100, 200, 510) ist.

2. System (300, 500) nach Anspruch 1, wobei die mindestens eine Eigenschaft des Belüftungssystems eine Gebläsedrehzahl ist.

## Revendications

1. Système (300, 500) comprenant :
un casque (100, 200, 510) configuré pour être porté sur une tête d'un utilisateur pour protéger l'utilisateur ;
le casque comprenant :
un dispositif cranio-cervical de soudage (110) configuré pour être porté sur une tête d'un utilisateur pour protéger l'utilisateur au cours d'un processus de soudage ;
un appareil d'avertissement intelligent (120, 520) intégré au dispositif cranio-cervical de soudage (110) ;
un système de ventilation ;
un dispositif de surveillance d'aérosol externe (310) couplé de manière opérationnelle au casque (100, 200, 510) ; et
ledit appareil d'avertissement intelligent étant configuré pour :
interfacer en communication avec le dispositif de surveillance d'aérosol externe (310) pour recevoir des données de sortie d'échantillon d'air depuis ledit dispositif de surveillance d'aérosol externe ;
l'appareil d'avertissement intelligent (120, 520) étant configuré pour communiquer avec le système de ventilation, en commandant ainsi au moins une propriété du système de ventilation,
dans lequel ledit appareil d'avertissement intelligent (120, 520) inclut un processeur (125) configuré pour traiter lesdites données de sortie d'échantillon d'air reçues depuis ledit dispositif de surveillance d'aérosol externe pour générer un signal utilisé pour commander l'au moins une propriété du système de ventilation,
dans lequel ledit appareil d'avertissement intelligent (120, 520) inclut en outre une interface utilisateur (121) connectée de manière opérationnelle audit processeur (125) et configurée pour permettre à un utilisateur de sélectionner un mode de fonctionnement d'avertissement prédéfini incluant des points de consigne de niveau d'exposition présent et se corrélant à un processus de soudage particulier en incluant des paramètres correspondant audit processus de soudage particulier,
dans lequel ladite interface utilisateur (121) est configurée pour permettre à un utilisateur de régler un seuil d'exposition, dans lequel un état de l'au moins une propriété est commandé sur la base du seuil d'exposition, et dans lequel le seuil dépend d'un type de contaminants en suspension dans l'air,
**caractérisé en ce que**
le système de ventilation est un respirateur d'épuration d'air motorisé (360) pour le casque de soudage (100, 200, 510).

2. Système (300, 500) selon la revendication 1, dans lequel l'au moins une propriété du système de ventilation est une vitesse de ventilateur.
